# EUROPEAN PATENT SPECIFICATION

(11) **EP 2 292 729 B1**
(45) Date of publication and mention of the grant of the patent: **23.04.2014**
(21) Application number: 09773452.9
(22) Date of filing: 30.06.2009
(51) Int. Cl.: C12M 1/00, C12M 1/38, C12M 3/00, B01L 7/00, B01L 1/02

(54) **CONSTANT-TEMPERATURE EQUIPMENT**
VORRICHTUNG ZUR TEMPERATURKONSTANTHALTUNG
ÉQUIPEMENT À TEMPÉRATURE CONSTANTE

(30) Priority: 01.07.2008 JP 2008172491
(43) Date of publication of application: 09.03.2011
(73) Proprietor: Rorze Corporation, Hiroshima-ken 720-2104 (JP)
(72) Inventor: YAMASHITA, Seishi, Fukuyama-shi Hiroshima 720-2104 (JP)
(74) Representative: Kramer - Barske - Schmidtchen
(86) International application number: PCT/JP2009/061902
(87) International publication number: WO 2010/001874

(56) References cited:
- WO-A1-03/008103
- WO-A1-03/008103
- JP-A- 11 166 555
- JP-A- 2007 132 467
- JP-A- 2007 319 119
- JP-U- 5 088 298
- JP-U- 5 096 564
- JP-U- 61 182 300
- US-A1- 2007 281 351

## Description

### FIELD OF THE INVENTION

The invention relates to constant-temperature equipment for maintaining at least an even temperature.

### BACKGROUND OF THE INVENTION

Constant-temperature equipment has been made use of to culture or test microbes and cells and so on. The constant-temperature equipment is formed by providing with means for maintaining environmental conditions such as the temperature, the humidity and the density of carbon dioxide to a temperature-controlled chamber for containing many samples which are the subject of culturing and testing. Culturing and testing are carried out continuously for many hours, and during these processes, it is necessary to regularly grasp conditions of each sample. Therefore, it is indispensable to regularly take out the sample from the temperature-controlled chamber to examine and analyze. Therefore, many of constant-temperature equipments introduced recently are equipped with memory means, arithmetic means and a conveyance mechanism to automate their process. The equipment has a function for automatically carrying a container having a sample out and in, delivering it to the processes for examining and analyzing, and handling the sample conditions. According to this, culturing and testing for the long term can be efficiently carried out.

### PRIOR ART

### PATENT LITERATURE

Patent literature 1 : Japanese Patent Laid Open Publication No. 2001-172
Patent literature 2 : Japanese Patent Laid Open Publication No. 2005-500522

WO-A-03/008103 relates to laboratory incubators employed to store chemical compounds, for example, wherein a robot located outside an incubator is used to open individual doors of the incubator for placing such chemical compounds.

US 2007/02811351 relates to culture systems to culture cells, wherein a conveyance mechanism for placing culture cells within an incubator is disposed within the culture apparatus.

### DISCLOSURE OF THE INVENTION

### PROBLEMS TO BE SOLVED BY THE INVENTION

However, as for conventional constant-temperature equipment with an automatic conveying function, various problems are caused on carrying out culturing and testing because there are mechanical and electrical components in a temperature-controlled chamber. The conventional constant-temperature equipment is affected by environments such as the temperature and the humidity during culturing to cause high frequency of trouble. Even if repair or maintenance is carried out, much time is required and culturing and testing start late because its structure is complicated. Besides, when a trouble occurs in the middle of culturing or testing, culturing and testing may be greatly affected on the reliability of themselves.

On the other hand, miscellaneous germs must be removed from the inside of the temperature-controlled chamber before starting culturing and testing. Because new cells or microorganisms to be next cultured and tested are affected and the reliability of culturing and testing for the long time may be harmed in case miscellaneous germs in the air or cells and microorganisms previously used in culturing and testing are left in the temperature-controlled chamber. Accordingly, in the conventional incubator, sterilizing treatments such as ultraviolet sterilization or dry sterilization have been carried out, or sterilization has been carried out by wiping off with medical solution.

However, various problems occur on carrying out the above-mentioned sterilizing treatments with the conventional control-temperature equipment having the automatic conveying function. For example, in the ultraviolet sterilization, ultraviolet rays do not reach the shadow of a structure, and therefore, they must be applied from various directions many times. In case of the dry sterilization, electric components and sealing members are damaged at high temperature from 150 °C to 180 °C. Accordingly, the dry sterilization has been carried out at low temperature which does not damage each component, or the inside has been taken apart to wipe off with the medical solution.

In case of the constant-temperature equipment with a small opening only for automatic conveyance to take the container in and out, even if a door is provided to keep the inside of the chamber isolated from the outside, it must be opened when taking the container in and out. The atmosphere inside the temperature-controlled chamber is affected by the outside environment to decline the reliability of culturing and testing. The culturing and testing are carried out over the long time, and therefore, the above-mentioned problems are strongly hoped for a solution.

The invention is to be thought up to effectively solve the above-mentioned problems.

### MEANS TO SOLVE THE PROBLEM

The constant-temperature equipment of the invention comprises a temperature-controlled chamber, a slide frame, a plurality of shielding plates and a conveyance mechanism. The temperature-controlled chamber has a sample shelf having a plurality of shelves provided to the inside, having an opening portion which is opened to the outside at position of the shelves of the sample shelf. The slide frame is provided along a side edge of the opening portion. The shielding plates are guided to the slide frame, closing the opening portion. Each shielding plate closes and opens an interval between adjoining shielding plates while being guided to the slide frame. The conveyance mechanism has detachable pieces which are attached removably to the shielding plates and moves on the shielding plates along the side edge of
the opening portion. The conveyance mechanism is attached removably on an external wall to a position facing the shielding plates.

The temperature-controlled chamber of this invention is a chamber for accommodating the sample, being a part of the constant-temperature equipment with a function for regulating the gas density such as moisture, oxygen, nitrogen, carbon dioxide, besides enabling to keep indoor temperature constant at least. Specially, the constant-temperature equipment of this invention is the most suitable for a culture vessel to be used in biology.

### EFFECTS OF THE INVENTION

According to the above-mentioned structure, the conveyance mechanism can access to the inside of the temperature-controlled chamber within the range of a pass box having required capacity, and therefore, it is possible to maintain a stable culturing and testing environment without affecting an internal environment in which the temperature and the humidity are kept constant in carrying the container in and out.

According to the structure wherein the shielding plates are opened and closed by sliding and ones except for a required opening portion are closed, it is possible to maintain the stable culturing and testing environment without appearing any current in the temperature-controlled chamber at the time of opening and closing.

### BRIEF DESCRIPTION OF THE DRAWINGS

Fig. 1 is a view for explaining constant-temperature equipment of an embodiment of the invention.
Fig. 2 is a view of a removable part of constant-temperature equipment of an embodiment of the invention.
Fig. 3 is a section view of constant-temperature equipment of an embodiment of the invention.
Fig. 4 is a section view for explaining an embodiment of conveyance mechanism.
Fig. 5 is a view for explaining a physical relationship between conveyance mechanism and a conveyed article.
Fig. 6 is a view for explaining the proper procedure for conveying a conveyed article with conveyance mechanism.
Fig. 7 is a view for explaining engaging means of shielding means.
Fig. 8 is a view for explaining a cross section of a shielding plate.
Fig. 9 is a view for explaining a form of magnets arranged on sample table drive mechanism.
Fig. 10 is a view for explaining a form of magnets arranged on a sample table.
Fig. 11 is a view for explaining an example of conveyance mechanism.
Fig. 12 is a view for explaining another example of conveyance mechanism.
Fig. 13 is a view for explaining a water saving portion in an example, which is provided on a floor of a temperature-controlled chamber.
Fig. 14 is a view for explaining a configuration of magnets.
Fig. 15 is a view for explaining a drive means using electro-magnets.
Fig. 16 is a view for showing another example of a pass box 53.
Fig. 17 is a view for explaining working of a dome.

### EXPLANATION OF REFERENCED NUMERALS

- 1: constant-temperature equipment
- 2: a box
- 3: a door
- 4: a sample shelf
- 5: a sample table
- 6: sample table drive mechanism
- 7: a large opening
- 8: a small opening
- 9: a shielding plate
- 10: a slide frame
- 11: conveyance mechanism
- 12: travel means
- 13: an external wall for temperature-controlled chamber
- 14: a sample container
- 15: a temperature-controlled chamber
- 16: an internal wall
- 17: a space
- 18: a base board
- 19: a rolling body
- 20: a locational tool
- 21: a driven magnet
- 22: heat insulating material
- 23: a driving plate
- 24: a gear motor
- 25 (25a, 25b): a pulley
- 26: a belt
- 27: a magnetic housing
- 28: a spacer
- 29: a bearing
- 30: a driving magnet
- 31: a circular table
- 32: a shield
- 33: a finger
- 34: a spacer
- 35: a slewing table
- 36: a gear motor
- 37a: a first arm
- 37b: a second arm
- 38: a bearing
- 39: a base shelf
- 40: a plate
- 41: a gear
- 42: a slewing motor
- 43: a gear
- 44: a shield shelf
- 45: a fixing block
- 46: a block
- 47: a top plate
- 48: an engaging pin
- 49: a sensor
- 50: an opening/closing door
- 51: a rotary source
- 52: a side wall
- 53: a pass box
- 54: an elevator gear motor
- 55: a pinion gear
- 56: a slide guide
- 57: a move terminal
- 58: an elevator base
- 59: a rack gear
- 60: a rack base
- 61: a solenoid
- 62: a micro-opening
- 63: a bracket
- 64: a bracket
- 65: an upper shielding plate
- 66: a long hole
- 67: a slewing shielding plate
- 68: a fixed shielding plate
- 69: a pulley
- 70: a screw hole
- 71: a shielding plate
- 72: an extract hole
- 73: a bearing
- 74: a pulley
- 75: a belt
- 76: an engaging hole
- 77: an engaging unit
- 78: a bracket
- 79: a water saving portion
- 80: an electro-magnet unit

### PREFERRED EMBODIMENT OF THE INVENTION

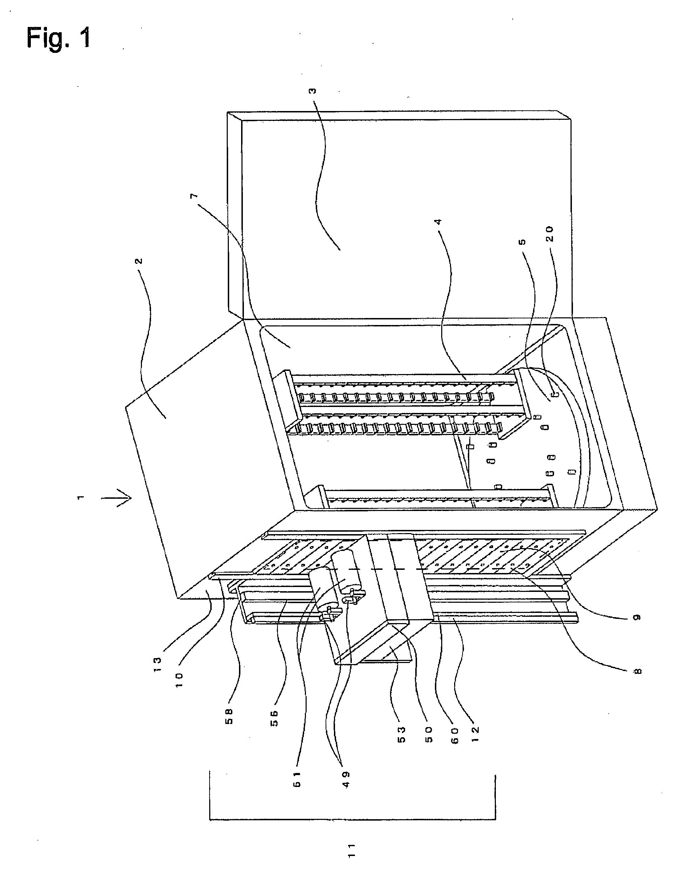

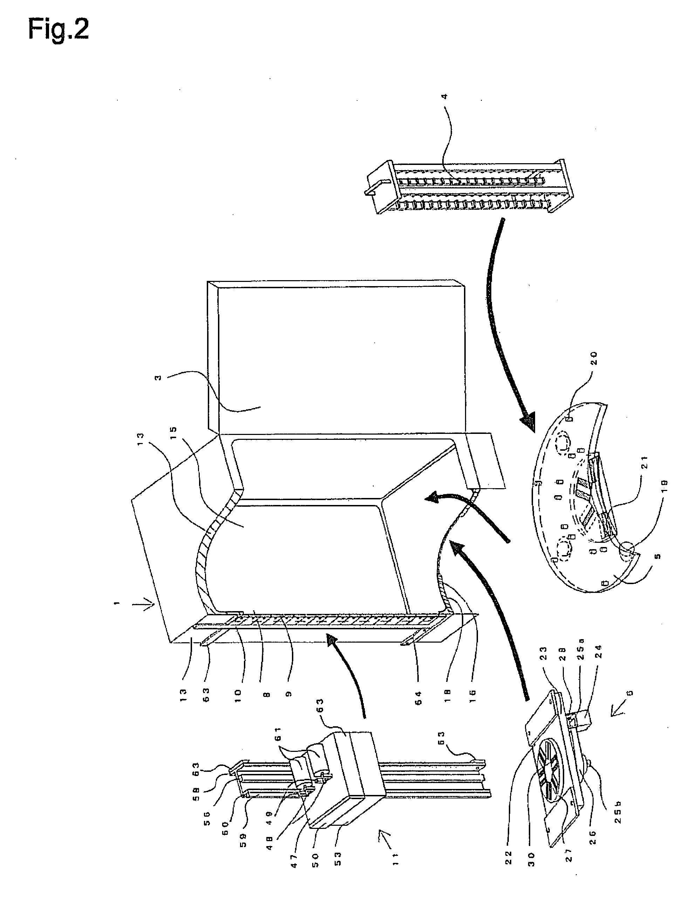

An embodiment of the invention will be explained with reference to following drawings. Fig. 1 is a perspective view of constant-temperature equipment 1 of this embodiment, and Fig. 2 illustrates a state for separating parts removable from the constant-temperature equipment 1. The constant-temperature equipment 1 comprises a box 2, a door 3, a sample table 5 for placing a sample shelf 4, and sample table drive mechanism 6 for rotating the sample table 5. The box 2 has two openings. One is a large opening 7 for carrying the sample shelf 4 or the sample table 5 in and out, which is used also to carry out maintenance. The other is a small opening 8 for carrying a sample container 14 loaded on each shelf of the sample shelf 4 in and out. The small opening 8 forms a liner opening having a length for exposing all shelves of the sample shelf 4 to the outside of the constant-temperature equipment 1. The large opening 7 has the door 3, which blocks the opening, provided through hinges so as to be freely opened and closed.

A plurality of shielding plates 9 are vertically piled on the small opening 8 movably along a slide frame 10 in accordance with the number of shelves in the vertical direction of the sample shelf 4. Each shielding plate 9 has an area enough to individually carry the sample container 14 in and out. The slide frame 10 guides the shielding plates 9 in the vertical direction. Here, the temperature-controlled chamber 15 forms a space isolated from the outside by shielding the small opening 8 with the shielding plates 9.

Conveyance mechanism 11 having a function for carrying the sample container 14 in and out is attached removably to the position facing the shielding plates 9. In addition, the conveyance mechanism 11 is gone up and down by travel means 12 of a part of the conveyance mechanism 11. According to this, by combining various workings of the drive means provided to the conveyance mechanism 11, it is possible to open and close the shielding plates 9, and to hold up, carry in and out and place the sample container 14. Furthermore, the conveyance mechanism 11 is attached removably on an external wall 13 of the constant-temperature equipment 1, which can be removed from the constant-temperature equipment 1.

Means for keeping an environment in the temperature-controlled chamber 15, which is not illustrated in drawings, is provided in a space 17 formed by an internal wall 16 of the temperature-controlled chamber 15 and the external wall 13. Environment conditions such as the temperature inside the temperature-controlled chamber 15 and the density of carbon dioxide therein are kept in required conditions.

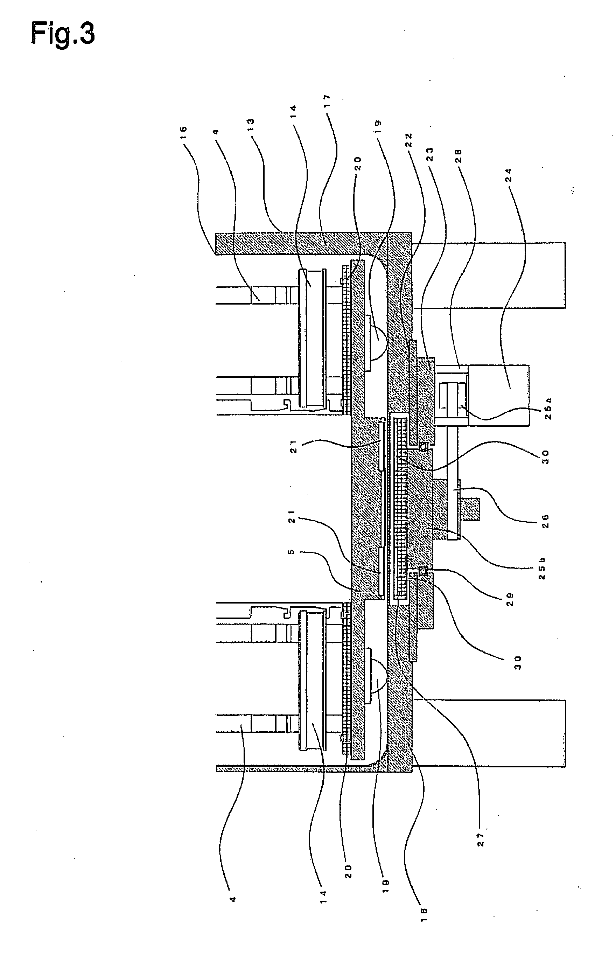

Next, internal structure of the box 2 and the sample table drive mechanism 6 will be explained with reference to Fig. 2 and Fig. 3. The internal wall 16 and the external wall 13 are fixed on a base board 18. The internal wall 16 has a function for perfectly blocking except for the large opening 7 and the small opening 8. The temperature-controlled chamber 15 contains the sample container 14 including a sample, the sample shelf 4 for putting a plurality of sample containers 14, and the sample table 5 for placing a plurality of sample shelves 4.

The sample table 5 has rolling bodies 19 such as ball casters fitted on the back side of the bottom at regular intervals so as to be installed movably on the floor inside the temperature-controlled chamber 15. In addition, the sample table 5 has driven magnets 21 fixedly placed on the back side so as to follow the workings of driving magnets 30 which are provided to the sample table drive mechanism 6 as a magnetic generator. Although this invention is subject to the rolling bodies 19, even in another example wherein an assembly made with materials having low frictional resistance instead of a rolling member is fitted on the bottom, the same effects can be obtained. Moreover, the sample table 5 has a locational tool 20 provided on the top, enabling to position in placing the sample shelf 4.

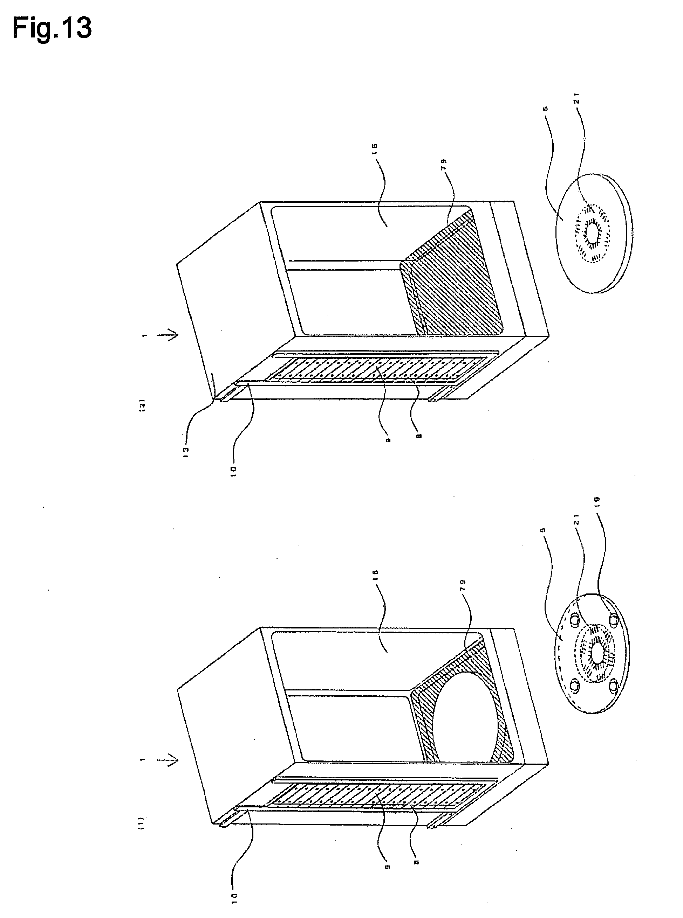

In examples shown in Figs. 13(1) and 13(2), a water saving portion 79 for keeping the humidity is provided. In Fig. 13(1), a cavity for saving demineralized water is formed by lowering the floor on which the rolling body 19 works. In addition, the temperature-controlled chamber 15 saves demineralized water throughout the bottom. In the example of Fig. 13(2), the temperature-controlled chamber 15 has a function for keeping the humidity by filling the bottom with the demineralized water. In this case, the water resisting property is improved by using the rolling body 19 made of resin or by covering all the bottom of the sample table 5 with low frictional resistance members.

The sample table drive mechanism 6, which drives the sample shelf 4 in an optional direction and at optional speed, is installed on the back side of the bottom of a base board 18 through heat insulating material 22. According to this, even the inside of the temperature-controlled chamber 15 is kept at high temperature, components for constituting the sample table drive mechanism 6 are hardly affected by the heat in the temperature-controlled chamber 15. The sample table drive mechanism 6 comprises a driving housing unit 23 like a plate, a gear motor 24, a pulley 25a, 25b, a belt 26 and a magnetic housing 27. The gear motor 24 is installed on the driving housing unit 23 through a spacer 28. The sample table drive mechanism 6 has all of the components implemented on the driving housing unit 23, being attached removably to the temperature-controlled chamber 15 as one body by fitting the driving housing unit 23 to the outside of the base board 18 with screws. The driving housing unit 23 can be also fixed with bolts. In case a screw with a hand grip, it is possible to easily carry out maintenances.

The gear motor 24 is electrically connected to a not-illustrated control assembly to set up parameters of the workings by input means like a keyboard. The pulley 25a is fixed to the axis of rotation of the gear motor 24. The rotary force of the gear motor 24 is transmitted to the pulley 25b by the belt 26. Here, the pulley 25b is rotatitively assembled to the driving housing unit 23 through bearings 29.

The pulley 25b has the magnetic housing 27 fixing a plurality of driving magnets 30 installed. Rotations of the motor are transmitted to the magnetic housing 27 at a fixed speed ratio through the pulleys 25a, 25b and the belt 26. Besides, the driving magnets 30 are fixed to the magnetic poles of the driven magnets 21 fixed on the bottom of the sample table 5 in a combination so that they pull against each other. According to this, the workings of the magnetic housing 27 are transmitted to the sample table 5 through the magnetic force to operate the sample table 5. In this case, the base board 18 and the walls of the temperature-controlled chamber 15A are formed by materials having no magnetism or poor magnetism. In addition, according to this structure, when bringing out the sample table 5 from the temperature-controlled chamber 15 to the outside, it can be brought out by merely picking up with one hand without tools.

There is no structure on the bottom of the temperature-controlled chamber 15. The positioning member is not also provided. Therefore, means for positioning in placing the sample table 5 becomes a problem. However, the sample table 5 can be located at the almost same position with before it is detached because it is introduced to the sample table drive mechanism 6 by mutual magnetic force of the magnets arranged on the magnetic housing 27 of the sample table 5 and the sample table drive mechanism 6. Besides, a self-aligning action works because the sample table 5 rotates by following rotary workings of the magnetic housing 27. That is, the sample table 5 can be reinstalled as keeping high positional repeatability by being lead so that the rotational center axis passes along the same line with a rotational center axis of the magnetic housing 27.

The sample table drive mechanism 6 uses a stepping motor or a servo motor as driving force. Accordingly, when the electric current is cut off or the gear motor 24 loses synchronism, positional data have been disappeared occasionally. Therefore, in this case, the position must be confirmed once more. The control assembly can catch the sample table 5 by providing means for detecting positions to the outside of the temperature-controlled chamber 15 and a detected substance to the sample table 5 as means for confirming positions.

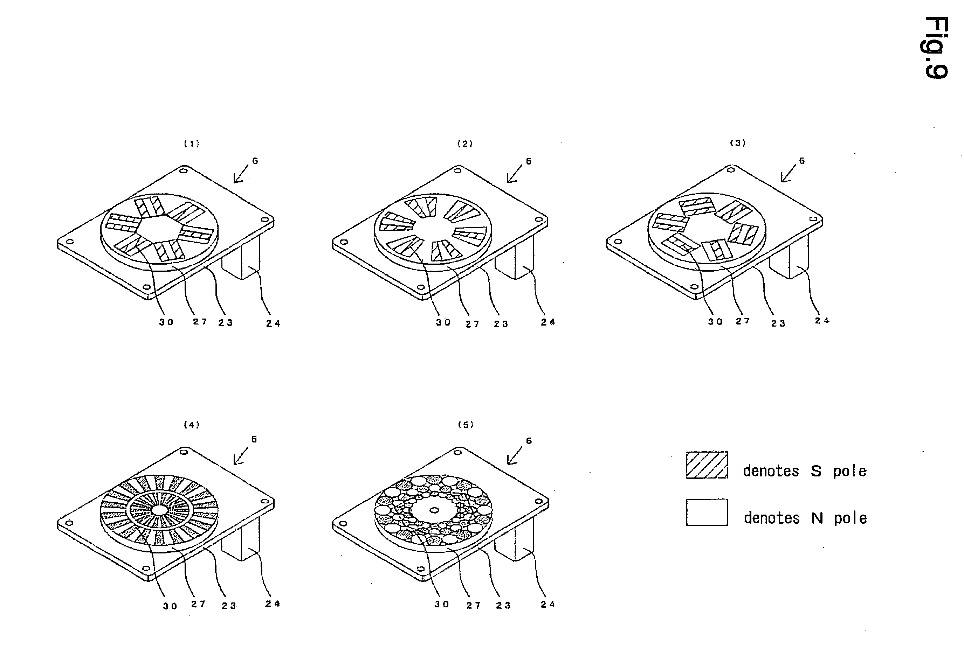

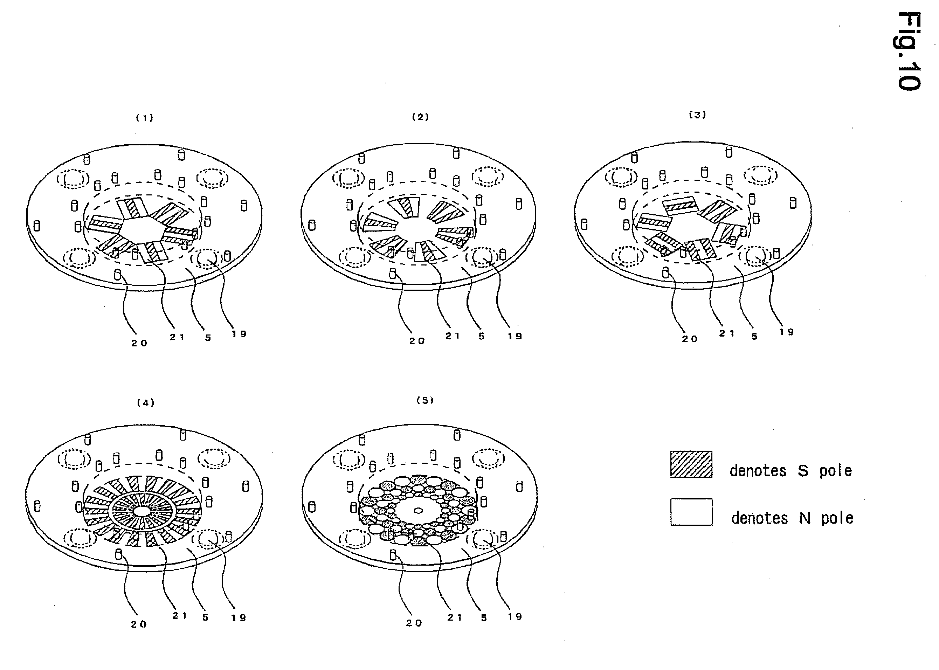

Next, the driven magnets 21, 30 will be explained. Fig. 9 is a perspective view of the sample table drive mechanism 6. Fig. 10 is a perspective view of the sample table 5, illustrating by peering through the top of the sample table. Both same numbers of Figs. 9(1) to 9(5) and Figs. 10(1) to 10(5) form a combination of driving force transmission. In the example of Figs. 9(1) and 10(1), rectangular magnets are used by combing magnets of three one set for alternately arranging N pole and S pole. There are two kinds of N-S-N and S-N-S as an arrangement of three magnets. In the example, all magnets of the sample table 5 and the magnetic housing 27 are arranged so as to pull against each other only when the sample table 5 rotates at a fixed angle to the magnetic housing 27.

According to this, even if the sample table 5 is removed from the temperature-controlled chamber 15 and reinstalled therein by hand, it can be replaced at the previous rotary position by rotating it therein and locating at the rotary position having the strongest magnetic force.

In Figs. 9(2) and 10(2), magnets of three one set as same as Figs. 9(1) and 10(1) form in a sector from the center of the magnetic housing 27. In Figs. 9(3) and 10(3), the sample table 5 is arranged so that the rotational center is close to the rotational center of the magnet housing 27 by dividing magnets of three one set in the rotational direction and the radial direction. Due to the arrangement of three one set magnetic, power transmission in rotational direction can be effectively obtained. That is, a self-aligning effect can be obtained more effectively.

In Figs. 9(4) and 10(4), stronger magnetic force can be obtained by installing magnets shaped in a sector on the whole magnetic housing 27. In Figs. 9(5) and 10(5), stronger magnetic force can be obtained by installing large and small circular magnets on the whole magnetic housing 27. The magnets have the form and the combination selected in various forms, numbers and arrangements. However, it goes without saying that it is desirable that the driven magnet 21 of the back side of the sample table 5 and the driving magnet 30 of the top of the magnetic housing 27 have common form, number and arrangement and besides the facing magnets have magnetic poles to pull against each other. Further, the driving magnet 30 has the magnetic force stronger by using a yoke.

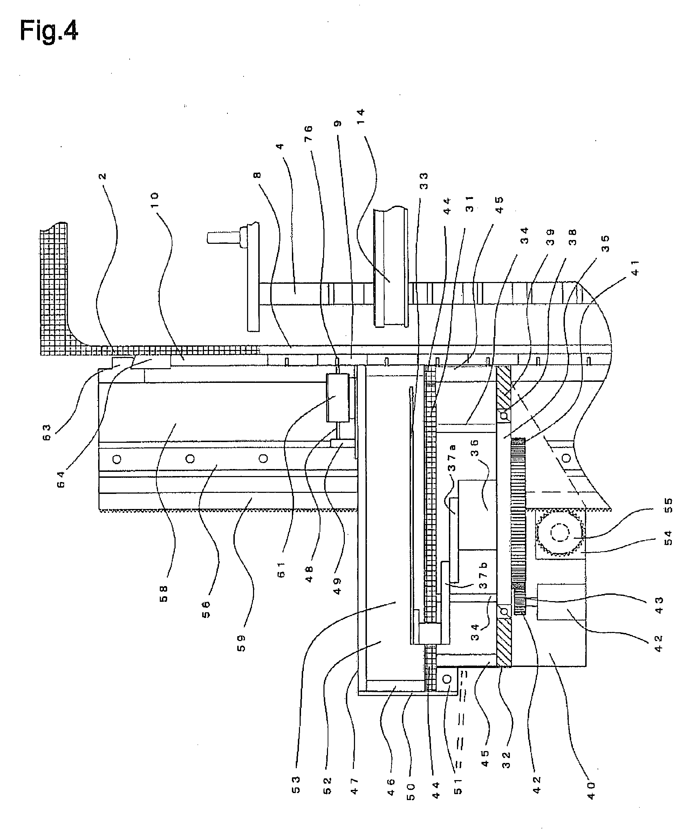

Next, the conveyance mechanism 11 will be explained with reference to Fig. 4. A gear motor 36 is fixed on a slewing table 35, and one end of a first arm 37a is installed to a rotational axis of the gear motor 36. The other end of the first arm 37a is combined to the base end of a second arm 37b, and the other end of the second arm 37b is combined to the base end of a finger 33. Here, the first arm 37a and the second arm 37b constitute an arm mechanism for advancing and backing the finger 33. The gear motor 36 has rotary motions transmitted to advance and back the finger 33 at a fixed speed ratio through the first arm 37a and the second arm 37b by installing them with a fixed speed ratio.

The slewing table 35 and a circular table 31 are fixed with spacers 34. The slewing table 35 is rotatively installed on a base shelf 39 through bearings 38 to form slewing mechanism. The base shelf 39 is fixed on a plate 40. The slewing table 35 is provided with a gear 41 for engaging with a gear 43 which is fixedly fitted to an axis of a slewing motor 42 which is fixed on the plate 40. According to this, the circular table 31 fixed on the slewing table 35, the gear motor 36, and the first arm 37a, the second arm 37b and the finger 33 fitted to the gear motor 36 are rotated by driving the slewing motor 42.

A shield shelf 44, which is provided with an opening having a diameter larger than the circular table 31, is fixed on the base shelf 39 through a fixing block 45 so as to be opposite the circular table 31. Accordingly, the circular table 31 and the shield shelf 44 serve as a partition between a pass box 53 for passing through the sample container 14 and the outside having the driving means.

The shield shelf 44 has a top plate 47 installed on the upper through a block 43. The top plate 47 has a detachable piece (an engaging pin 48 in the example) installed on the upper. The detachable piece is engaged with a concavity which is formed on the plane of the shielding plate 9, and advanced and backed by electromagnetic force of a solenoid 61. The engaging pin 48 pierces through the solenoid 61. When the engaging pin 48 is engaged with the shielding plate 9, it is advanced by the solenoid 61. When the engagement is released, the engaging pin 48 is backed by the solenoid 61. In addition, the engaging pin 48 has a sensor 49 provided to the rear to grasp the position for being advanced and backed.

The shield shelf 44 has an opening/closing door 50 fitted behind the finger 33 so as to freely open and close. The opening/closing door 50 is opened and closed by rotary source 51. Although the solenoid is used as the rotary source 51, a spring, an air-cylinder, or a motor can be also used. The shield shelf 44 and the top plate 47 are opposite the shielding plate 9 in a slight opening, having side walls 51 to the left and right sides. Accordingly, when the small opening 8 is opened, the pass box 53 serves as a buffer room for loosening a change of the atmosphere in the temperature-controlled chamber 15. Here, the pass box 53 comprises the shielding plates 9, the top plate 47, the opening/closing door 50, the side walls 52, the slewing table 35, the base shelf 39 and the shield 32. To heighten the isolating property, each contact portion has a not-illustrated rubber sealing member provided.

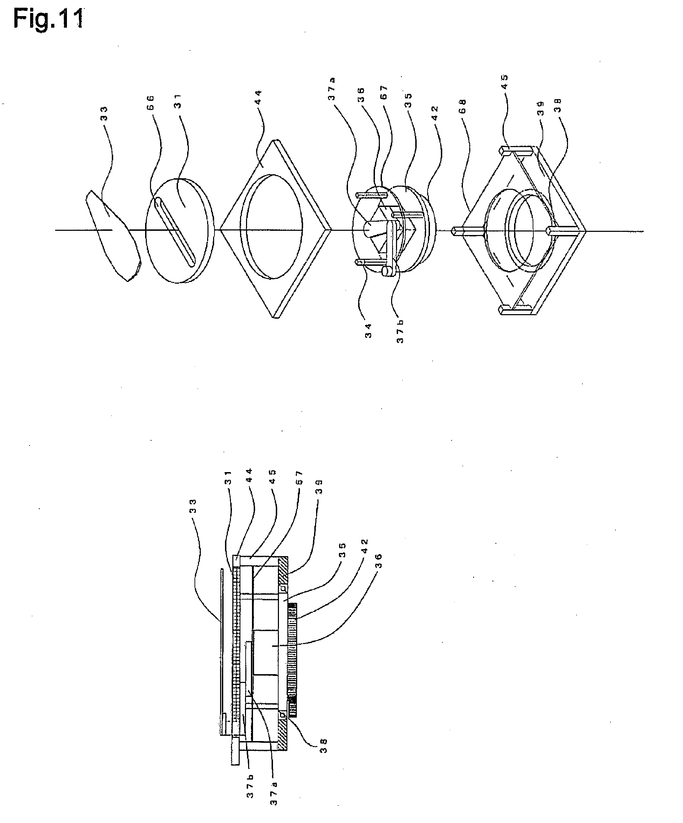

In the above-mentioned example, the circular table 31 has a long hole 66 along working of the finger 33 to work a coupling portion of the second arm 37b and the finger 33. However, according to this, the atmosphere having high temperature and high humidity in the temperature-controlled chamber 15 passes through the long hole 66 at the time when the shielding plates 9 are opened. In this case, the motor 36 may be broken. Therefore, as shown in Fig. 11 of the other example, the atmosphere can be prevented from entering the motor 36 by providing a slewing shielding plate 67 and a fixed shielding plate 38 between the motor 36 and the first arm 37a.

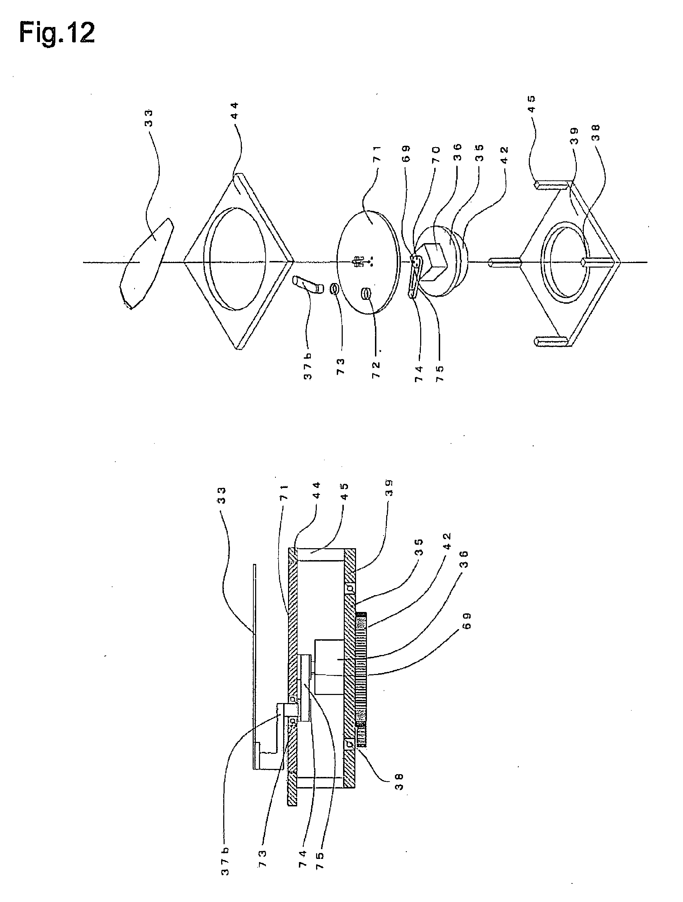

Furthermore, there is another example as shown in Fig. 12. Instead of the first arm 37a, a pulley 69 and a shielding plate 71 are concentrically fixed on the rotational axis of the motor 36. The shielding plate 71 has an extract hole 72 whose center is on the slewing track of the first arm 37a, and therein, the second arm 37b is inserted from the side of the finger 33 through a bearing 73. A pulley 75 is installed to connect the second arm 37b and the first arm 37a. When the rotary speed ratio of the pulley 69 and the pulley 74 is made same as the first arm 37a, the pulley 69 and the shielding plate 71 are rotated by the motor 36. In addition, rotations of the pulley 69 are transmitted to the pulley 74 through a belt 75 to advance and back the second arm 37b through the finger 33.

According to this method, the shield shelf 44 and the shielding plate 71 serve as a partition so that the mechanical structure below the second arm 37b can be isolated from the atmosphere having high temperature and high humidity in the temperature-controlled chamber 15. The isolating property is heightened by providing a bearing between the shielding plate 71 and the shield shelf 44.

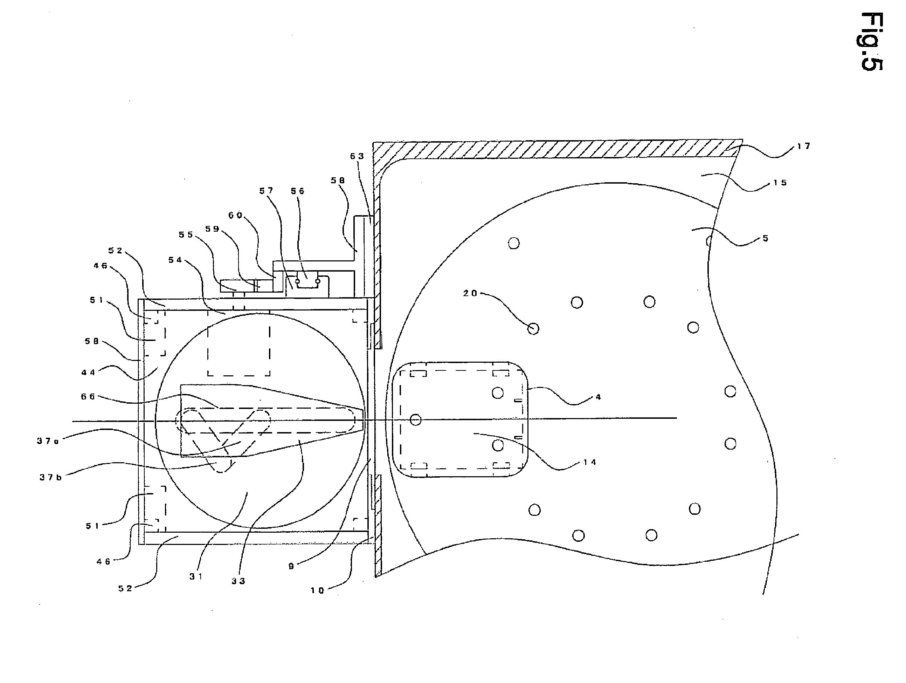

Next, the traveling means 12, which is a part of the conveyance mechanism 11, will be explained with reference to Figs. 4 and 5. The plate 40 has an elevator gear motor 54 for traveling installed, and the motor axis has a pinion gear 55 fixed. In addition, the plate 40 has a move terminal 57 of a slide guide 56 fixed on the back side, moving along the track of the slide guide 56 installed on an elevator base 58. The slide guide 56 of the elevator base 58 has a rack gear 59 having teeth for engaging with the pinion gear 55 installed through a rack base 60.

The elevator base 58 and the rack base 60 are detachably installed to the box 2 through brackets 63, 64 so that the engaging pins more than one engage in engaging holes 76 of the opposite shielding plates 9. Therefore, the engaging pins 48 are certainly engaged to the shielding plates 9 which are located at any position from the lowermost shelf to the uppermost shelf. Needless to say, the slide guide 56 and the rack gear 59 have a stroke enough to hold up one step for the shielding plates 9 positioning at every point from the lowermost shelf to the uppermost shelf. Although a rack-and-pinion method is adopted for elevating means of this example, in another example is provided elevating means according to a ball screw, and in further another example is provided elevating means according to a linear servo.

As shown in Fig. 5 which illustrates the equipment viewed from the top, at a home position, the shielding plate 9 crosses at right angle to the center line of the finger 33, and the sample table 5 is positioned so that the rotary center passes on a line for extending the center line of the finger 33. The finger 33 is advanced in the temperature-controlled chamber 15 through the small opening 8 by the gear motor 36, stopping at the lower tip position of the sample container 14 put on the sample shelf 4, slightly moving upwardly by rotating the rack gear 59, loading with the sample container 14, thereafter being backed by the gear motor 36. All input-output circuits of the gear motor 36, the slewing motor 42, an elevator gear motor 54, the solenoid 61 and the sensor 49 are electrically connected to a not-illustrated control assembly. An operator can optionally set up the operation with input means such as a key board and so on.

Accordingly, it is possible to bring out the sample container 14 put on the sample shelf 4 in the temperature-controlled chamber 15 or put the sample container 14 on the sample shelf 4 in the temperature-controlled chamber 15 from the outside automatically as the moving sequence set by the operator. The conveyance mechanism 11 can be attached removably from the box 2 as a boundary of the brackets 63, 64. In carrying out the dry sterilization in the temperature-controlled chamber 15, the conveyance mechanism 11 is taken out to avoid the effects of heat of the dry sterilization. According to this, the source of the trouble is decreased. Even if it is out of order, the maintenance can be carried out without affecting the inside of the temperature-controlled chamber 15. Therefore, it is unnecessary to break off culturing and testing expressly and it is possible to carry out efficient culturing and testing.

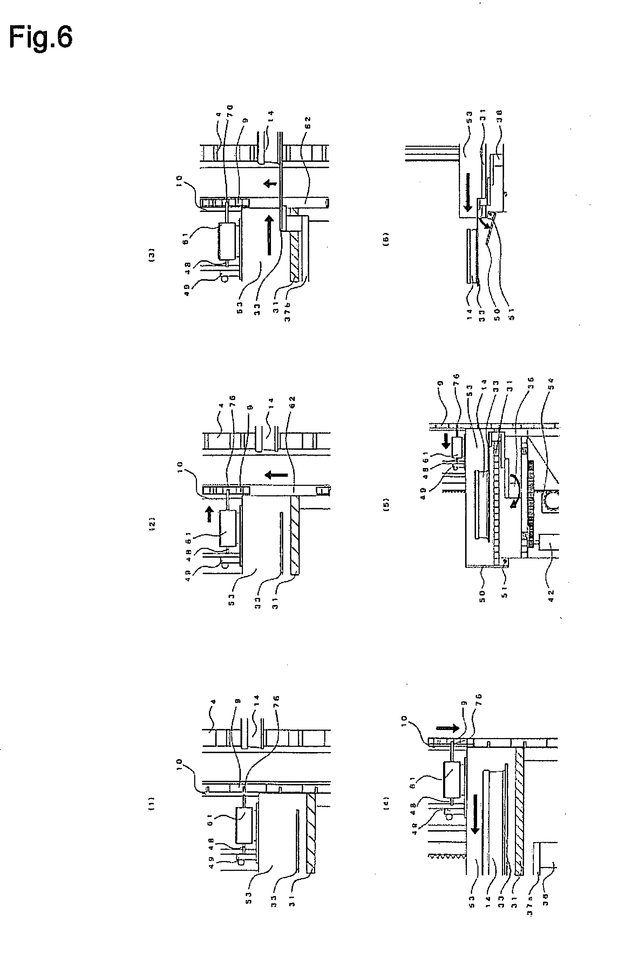

Next, a motion for carrying the sample container 14 of the conveyance mechanism 11 will be explained with reference to Figs. 6(1) to 6(6). Here, black arrows in the figures show a motion direction of each driving portion.

(1) As instructed by the operator, the sample shelf 4 for putting the sample container 14 is rotatively moved to the position where the finger 33 can be carried by the sample table drive mechanism 6 to be stopped. The engaging pin 48 of the conveyance mechanism 11 is elevated to the height of the engaging hole 76 of the shielding plates 9 corresponding to the indicated shelf of the sample shelf 4 by the travel means 12.

(2) Positional data of each motor are sent to the control assembly at any time. When the data reach the appointed position, a signal is sent from the control assembly, so that the solenoid 61 can move the engaging pin 48 toward the shielding plates 9. The control assembly judges according to on-off signals of the sensor 49 provided behind the engaging pin 48 if the engaging pin 48 fully engages in the engaging hole 76. It is possible to confirm the position of the engaging pin 48 by setting so that an optical axis of the sensor 49 can pass between the position where the rear end of the engaging pin 48 engages with the engaging hole 76 and the position insufficient for the engagement.

If the engaging pin 48 insufficiently shades, in other words, if the optical axis of the sensor 49 is kept shaded, it should be set up so that next motion is not carried out. When the engaging pin 48 is sufficiently engaged in the engaging hole 76, the control assembly orders so that the travel means 12 has the conveyance mechanism 11, the shielding plate 9 engaged with the engaging pin 48, and all shielding plates pilled up on the upper from the shielding plates 9 moving in a rising direction by an optional moving amount. According to this, upper shielding plates 65 over the shielding plate 9 engaged with the engaging pin 48 are moved in the rising direction within the slide frame 10 to produce a fine opening 62.

The moving amount is enough to produce the smallest opening possible to bring in and out the sample container 14, being one shelf of the sample shelf 4 for the maximum. According to this motion, the temperature-controlled chamber 15 is opened. However, since an opening isolated with the pass box 53 in the conveyance mechanism 11 occupies only the minimum volume necessary for conveying the sample container 14, the environmental change is held down to a minimum.

(3) When the elevator gear motor 54 finishes raising a shielding plate, the control assembly orders the gear motor 36 to advance the finger to an appointed position. Vertical positions of the finger 33 and the engaging pin 48 and the moving amount for opening the shielding plates 9 are determined according to the height of the sample container 14. When the finger 33 finishes forwardly moving to an appointed position below the sample container 14, the control assembly orders the travel means 12 to slightly rise to hold up the sample container 14.

(4) When the rise by the elevator gear motor 54 is finished and the finger 33 holds up the sample container 14, the control assembly orders the gear motor 36 to back the finger 33 to the position where it was and the sample container 14 is conveyed from the temperature-controlled chamber 15 to the pass box 53. When the finger 33 is backed to the position where it was and the sample container 14 is conveyed into the pass box 53, the control assembly orders the travel means 12 to lower the conveyance mechanism 11 to the position (1). According to this, the fine opening 62 is shielded and the temperature-controlled chamber 15 is formed in a closed space again.

(5) When the travel means 12 finishes moving the conveyance mechanism 11 downwardly, the control assembly orders the solenoid 61 to back the engaging pin 48 from the engaged state to the position (1). When the sensor 49 confirms that the backing working is finished, the control assembly orders the travel means 12 to go up or go down the conveyance mechanism 11 to the not-illustrated predetermined position of delivery of the sample container 14. After finishing moving to the position of delivery, the control assembly orders the slewing motor 42 to rotate a rotary portion in the opposite direction of 180 degrees.

(6) After the slewing working is finished, the control assembly orders the rotary source 51 of the opening/closing door 50 to open the door. After receiving a signal that opening the door is finished, the control assembly orders the gear motor 36 to advance the finger 33 to an appointed position of delivery. When the finger 33 advances to the position of delivery, the control assembly orders the elevator gear motor 54 to slightly lower the finger 33 on a not-illustrated table and to load the sample container 14 thereon. After the delivery is finished, the control assembly orders the gear motor 36 to put the finger 33 back where it was. After the working is finished, the control assembly orders the gear motor 36 to close the opening/closing door 50. According to this, the pass box 53 again forms a closed space, and then a series of conveyance working is complete.

According to this opening working, the pass box 53 is open to the outside. However, since the volume of the pass box 53 is minimized as much as possible, it is possible to minimize atmosphere replacement with the outside. That is, leakage to the outside of bacteria which floats in the temperature-controlled chamber 15 can be minimized, and inflow of miscellaneous germs from the outside into the temperature-controlled chamber 15 can be also minimized.

As the above-mentioned, the sample container 14 is carried out in order. In carrying in it, each shelf from (1) to (6) can be performed on the contrary.

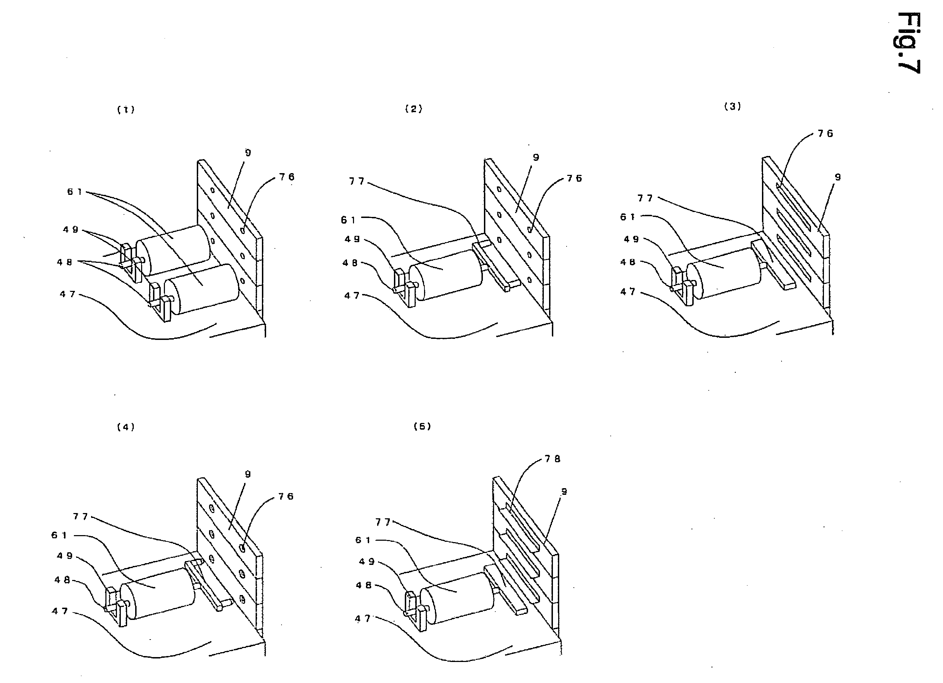

As to engage in an engaging hole 76 which is provided to the shielding plates 9, five kinds of forms as shown in Figs. 7(1) to 7(5) were carried out. In this example of Fig. 7(1), the engaging hole 76 has two engaging pins 49, two solenoids 61 and two sensors 49 provided. In Fig. 7(2), the engaging pin 48 has an engaging unit 77 provided to the tip and the solenoid 61 drives with one. In Fig. 7(3), the engaging hole 76 and the engaging unit 77 are respectively shaped rectangular, thereby enabling to reduce the number of components in comparison with the case in Fig. 7(1). In Fig. 7(4), the engaging hole 76 is shaped tapered, thereby enabling to assure the engagement still more. In Fig. 7(5), instead of the engaging hole 76, a bracket 78 is provided, thereby enabling more assured engagement in comparison with the cases in Figs. 7(1) to 7(4). Although the position for engaging the engaging pin 49 to the engaging hole 76 of each shielding plate 9 is determined by a method for inputting instructing data to the control assembly, the position of the engaging hole 76 can be detected by installing a sensor.

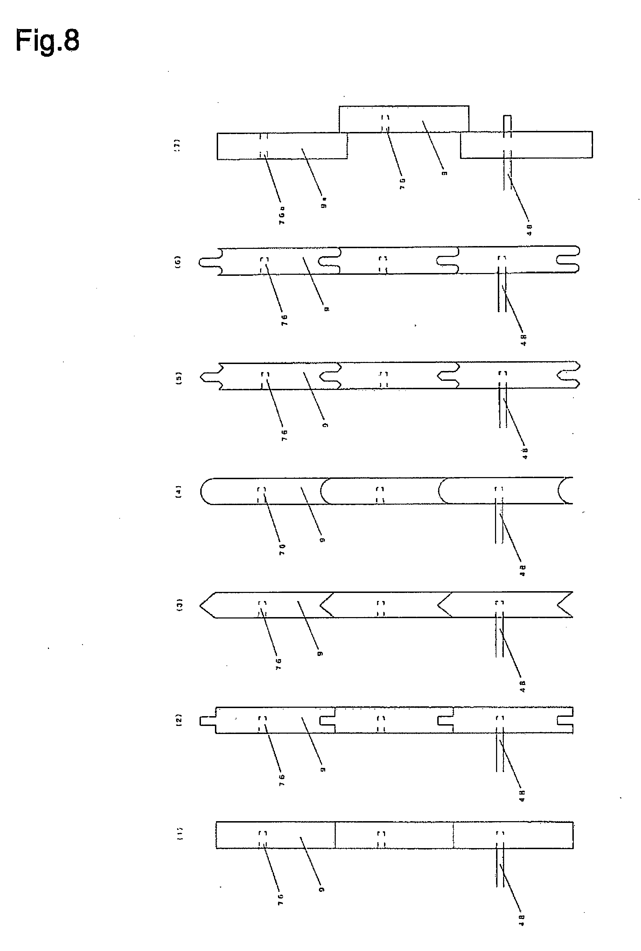

Next, sectional form of the shielding plate will be explained with reference to Figs. 8(1) to 8(7). Fig. 8(1) illustrates a section of the shielding plate 9 of this example, whose form is easy to be machined most. However, each section in Figs. 8(2) to 8(7) is adopted to enhance the airtightness. In Fig. 8(2), making unevenness for the top and the bottom of the shielding plate 9 enables to increase contact area between both shielding plates 9 to enhance the airtightness. In Figs. 8(3) and 8(4), the repeat accuracy in opening and closing is enhanced by inclining the interfitted portion. In case of Figs. 8(5) and 8(6), the airtightness related to Fig. 8(2) and the repeat accuracy each related to Fig. 8(3) and Fig. 8(4) are compatibly enhanced, wherein the uneven portions are inclined or rounded to be easily interfitted.

In an example of Fig. 8(7), the shielding plates 9 are arranged in two rows, whose parts contact with different shielding plates 9 one another. The engaging hole 76a of the shielding plate 9a, which is in front of the engaging pin 48, forms a through hole. The engaging pin 48 has a stroke enough to reach the engaging hole 76 of the shielding plate 9 which is arranged backward. According to this structure, open area equal to Figs. 8(1) to 8(7) can be gained by moving more few shielding plates. In addition, the shielding plates 9 are made members which are attracted by magnetic force in enhancing the airtightness in the examples of Figs. 8(1) to 8(7). During closing, the shielding plates 9 are adsorbed by the contacting slid frame 10 with electromagnets. In opening the small opening 8, the electromagnets have the electricity intercepted.

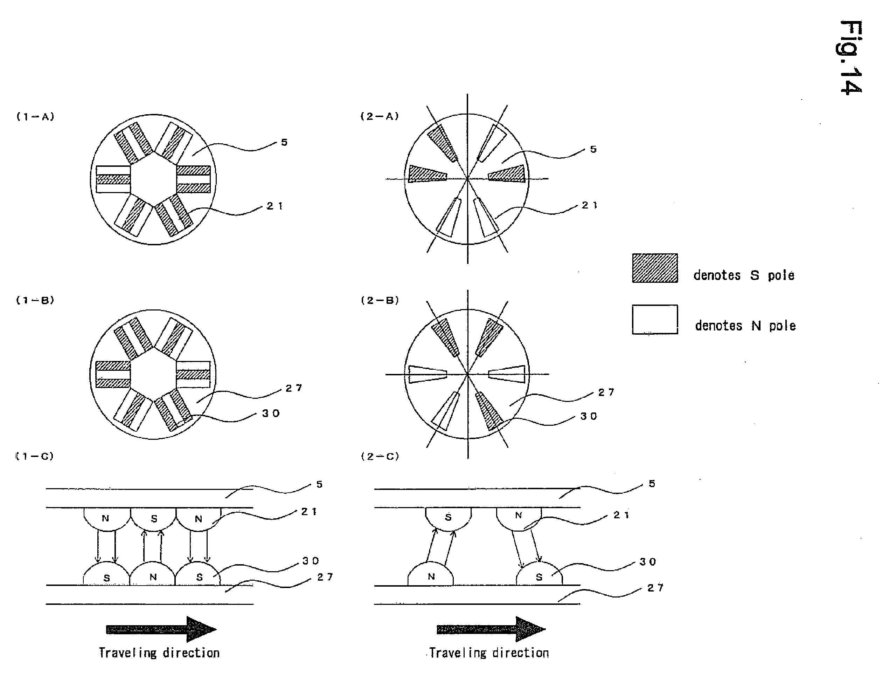

The driven magnets 21 on the back of the table 5 and the driving magnets 30 arranged on the magnet housing 27 will be explained with reference to Fig. 14. Each of Figs. 14(1-A) to 14(1-C) shows the arrangement of the magnets in this example, Fig. 14(1-A) is a view of the sample table 5 seen from the back side, and Fig. 14(1-B) is a view of the magnet housing 27 seen from the top side. Fig. 14(1-C) illustrates a vertical section of the magnets facing each other. In a stationary state, the opposite magnets pull against each other. When the magnet housing 27 is moved in a moving direction, the sample table 5 is moved by two forces which are the pulling force between the opposite magnets and the repulsion between adjacent magnets having the same magnetic polarity.

There are another example of Figs. 14(2-A), 14(2-B) and 14(2-C). Fig. 14(2-A) is a view of the sample table 5 seen from the back side, and Fig. 14(2-B) is a view of the magnet housing 27 seen from the top side. In Fig. 14(2-B), six driving magnets 30 are arranged on the magnet housing 27 at the same configuration, and the opposite driven magnets 21 are arranged on the sample table 5 so as to be equally shifted to the configuration of the driving magnet 30. Fig. 14(2-C) illustrates a vertical section of the opposite magnets. Since the upper and lower magnets are arranged at the positions having equal magnetic force at a fixed interval without straightly facing, the magnetic force decreases in dispersion. Therefore, it is possible to obtain the equal magnetic force with a few magnets.

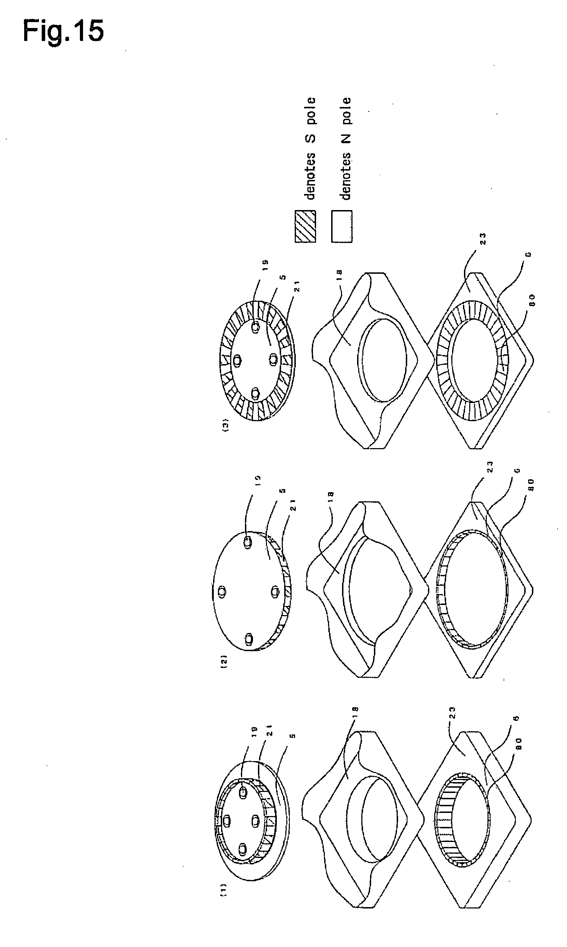

Next, an example of the sample table 5, which is driven without the motor 24, will be explained with reference to Fig. 15. In an example of Fig. 15(1), the sample table 5 has a cylinder on the bottom, and the driven magnets 21 (permanent magnet) each having different magnetic poles arranged on the peripheral wall of the cylinder by turns. In an example of Fig. 15(2), the sample table 5 is disk-like, and has the driven magnets 21 each having different magnetic poles arranged on the peripheral wall of the disk by turns. On the other hand, in the side of the sample table drive mechanism 6, an electro-magnet unit 80 is arranged on the inside of the peripheral wall so as to be opposite to the driven magnets 21. In an example of Fig. 15(3), the driven magnets 21 are arranged on the circumferentially on the plane in a vertical direction. On the other hand, in the side of the sample table drive mechanism 6, the electro-magnet unit 80 is arranged circumferentially on the plane so as to be opposite to the driven magnets 21. The sample table drive mechanism 6 has the electro-magnet unit 80 arranged on the inside of the peripheral wall or the electro-magnet unit 80 arranged circumferentially on the plane mounted on the driving housing unit 23. In addition, the sample table drive mechanism 6 is attached freely-removably to the temperature-controlled chamber 15 as one body by fitting the driving housing unit 23 to the base board 18 with the screws. In the examples of Figs. 15(1), 15(2), 15(3), the driving housing unit 23 is attached removably to the bottom of the base board 18, and the inside of the temperature-controlled chamber 15, in which the driven magnets 21 are included, is separated from the outside, in which the electro-magnet unit 80 is located, by the inner wall 16.

In all examples of Figs. 15(1), (2), (3), the electro-magnet unit 80, which are adjacent or located at intervals of the fixed number of magnets, are arranged in the driving housing unit 23 so as to respectively have opposite magnetic polarities. The sample table 5 is operated by shifting magnetic field which is generated by switching in order the magnetic polarity of the electro-magnet unit 80 through a control unit (not-illustrated) inside or outside the driving housing unit 23. Besides, there should be the driven magnets 21 which enough magnetic force is got to operate the sample table 5 even if they are not arranged for all circumference of the sample table 5. In addition, as the explanation about Fig. 14(2), in switching the magnetic field of the electro-magnet unit 80, the sample table 5 is surely moved by respectively arranging the electro-magnet unit 80 of the driving side and the magnets 19 of the driven side to the positions having balanced magnetic forces at equal intervals. Besides, the smoothness of the working of the sample table 5 and the accuracy of positioning are enhanced by increasing the number of electromagnets and making it small and by sizing the driven magnet 21 equal to the electromagnet.

In these examples, rotary magnetic field is generated as shifting magnetic field. However, in moving the sample table 5 linearly, liner shifting magnetic field is generated by arranging the electromagnets linearly.

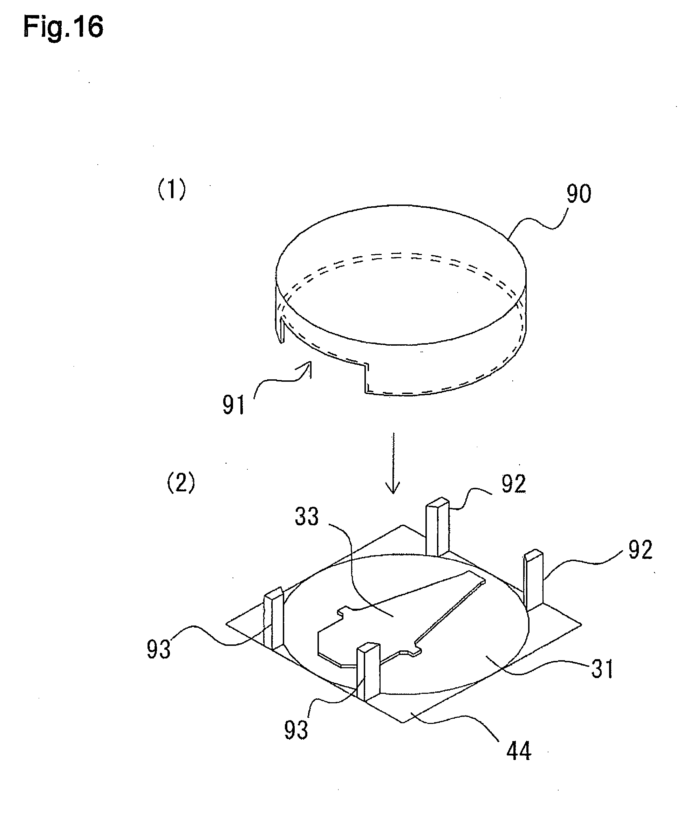

Fig. 16 is a view for showing another example of the pass box 53.
Fig. 16(1) illustrates a dome 90 which is located in the inside of the pass box 53. The dome 90 forms a cylinder having a ceiling portion, on which a window 91 is provided. The dome 90 is mounted so as to cover a circular table 31, constituting a so-called rotary door by rotating in a body with the circular table 31. Pair of spacers 92, 93, which are away in accordance with the size of the window, are provided to the side of the temperature-controlled chamber 15 and the opposite side on the shield shelf 44.

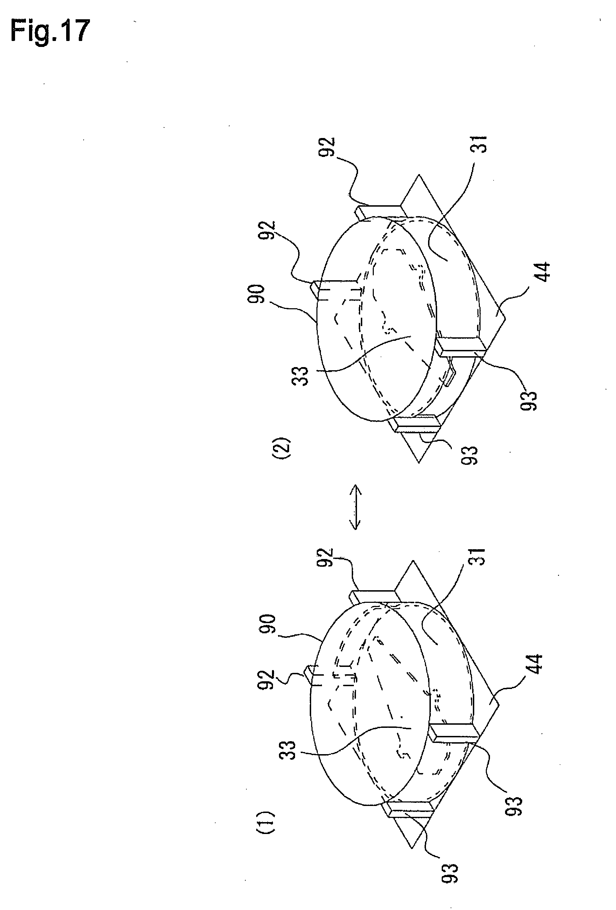

As shown in Figs. 17(1) and 17(2), when the window is positioned at the side of the temperature-controlled chamber 15 by the spacers 92, a space within the dorm 90 is not connected to a space within the pass box 53. In addition, according to the spacers 93, an atmosphere within the temperature-controlled chamber at the opposite side can minimize affecting on the external environment.

As explained in the above, the following embodiments can be carried out in this invention.
(1) In a constant-temperature equipment comprising a temperature -controlled chamber, means for controlling environment conditions in the temperature-controlled chamber, a sample table freely removably arranged in the temperature-controlled chamber, a sample shelf freely removably attached on the sample table, a sample table drive mechanism for driving the sample table from the outside of the temperature-controlled chamber, a large opening portion provided to one plane of the temperature-controlled chamber, a small opening portion provided to one side plane of the temperature-controlled chamber so that at least one sample container passes through, a conveyance mechanism for carry in and out at least one container through the small opening portion, and a control assembly for controlling the conveyance mechanism and the sample table drive mechanism, the sample table drive mechanism is freely removable and insertable outside the temperature-controlled chamber. Here, the sample shelf is to contain a plurality of containers accommodating the samples. The large opening portion is provided with a freely-opening/closing door to carry in and out the sample table and/or the sample shelf. The small opening portion is provided with a shielding plate which freely opens and closes.
(2) The conveyance mechanism can be attached removably to the external wall of the temperature-controlled chamber.
(3) The sample table has a rolling body or a low frictional member on a plane opposite to the sample table drive mechanism.
(4) The sample table drive mechanism has magnet generating means which is operated by driving source. The sample table has the driven magnets arranged at the positions corresponding to the magnet generating means through members for forming the temperature-controlled chamber. According to this, the driving force from the driving source is transmitted to the sample table by non-contact coupling based on the magnetic force passing through the members forming the temperature-controlled chamber.
(5) A permanent magnet is used for a driven magnet which is provided to the sample table, and also for magnet generating means which is provided to the sample table drive mechanism.
(6) A permanent magnet is used for a driven magnet which is provided to the sample table, and a direct current electromagnet is used for magnet generating means which is provided to the sample table drive mechanism.
(7) The sample table drive mechanism, which has no movable portions, has alternate current electromagnets or direct current electromagnets fixed. The sample table has the driven magnets arranged so as to be opposite to the alternate current electromagnets or the direct current electromagnets through the member forming the inside of the temperature-controlled chamber. The driving force, which is generated by a variation of phases of the alternate current electromagnets or the direct current electromagnets which are adhered on the back side of the members forming the inside of the temperature-controlled chamber, is transmitted to the sample table by the non-contact coupling based on the magnetic force passing thorough the walls of the temperature-controlled chamber.
(8) The sample table drive mechanism is attached removably on the back side of the temperature-controlled chamber. The sample table drive mechanism has the magnet generating means which is operated by the driving source. The sample table has the driven magnets arranged so as to be opposite to the magnet generating means through the member forming the temperature-controlled chamber. According to this, the driving force from the driving source is transmitted to the sample table by the non-contact coupling based on the magnetic force passing through the member forming the temperature-controlled chamber.
(9) The conveyance mechanism comprises a finger, an arm mechanism for advancing and backing the finger, slewing means for slewing the finger and the arm mechanism, and travel means for moving the slewing means vertically or laterally.
(10) The conveyance mechanism accommodates at least the finger and the container, and has a pass box in a route for carrying the container.
(11) In addition, the shielding plate opening and closing means is provided.
(12) The shielding plates more than one are arranged parallel to the travel means. The conveyance mechanism is provided with engaging means for the shielding plates to open and close them by moving workings of the travel means.
(13) The temperature-controlled chamber has a lengthwise opening on one side plane. A plurality of shielding plates is vertically pilled up inside two slide-guides which are provided on the external wall of the constant-temperature equipment outside the lengthwise opening. The conveyance mechanism lifts all the upper shielding plates including the opposite ones by engaging with the opposite ones. According to this, a small opening for passing at least one sample container is formed.
(14) The shielding plates are respectively opposite to the shelves of the sample shelf at the time when the small opening is closed.
(15) The shielding plates have either the engaging hole or the bracket engaged with the engaging means of the conveyance mechanism.

According to the above-mentioned construction, the constant-temperature equipment can rotate the sample table by having a turntable on the sample table drive mechanism and arranging either one kind of the permanent magnets or the direct current electromagnets circularly for the center on the turntable.
(16) The constant-temperature equipment comprises a temperature-controlled chamber, means for controlling environment conditions in the temperature-controlled chamber, a sample table freely removably arranged in the temperature-controlled chamber, a sample shelf freely removably attached on the sample table, sample table drive mechanism for driving the sample table from the outside of the temperature-controlled chamber, a large opening portion provided to one plane of the temperature-controlled chamber, a small opening portion provided to one side plane of the temperature-controlled chamber so that at least one sample container passes through, conveyance mechanism for carrying in and out at least one container through the small opening portion, and a control assembly for controlling the conveyance mechanism and the sample table drive mechanism. Here, the sample shelf is to contain a plurality of containers accommodating the samples. The large opening portion is provided with a freely-opening-and-closing door to carry in and out the sample table and/or the sample shelf. The small opening portion is provided with a shielding plate which freely opens and closes. In addition, the conveyance mechanism is outside the temperature-controlled chamber, being attached removably on the external wall thereof, besides having means for transmitting the driving force to the sample table by the non-contact coupling based on the magnetic force which passes through the walls of the temperature-controlled chamber for separating from the outside.
(17) The temperature-controlled chamber is a chamber for accommodating the sample, being a part of the constant-temperature equipment having the function for controlling the density of gas such as humidity, oxygen, nitrogen and carbon dioxide besides at least keeping a fixed indoor temperature. Specially, the constant-temperature equipment of this invention is most suitable for a culture tank which is used in biological field.
(18) The non-contact coupling based on the magnetic force can be carried out by combinations more than one among the permanent magnets and the permanent magnets, the permanent magnets and the direct current electromagnets, and the permanent magnets and the alternate current electromagnets. In addition, stronger magnetic force can be obtained by eliminating the dispersion of flux by respectively locating yokes to the magnets.
(19) The sample table drive mechanism has magnetic housing which rotates or linearly moves, having either one kind of the permanent magnets or the direct current electromagnets arranged on the magnetic housing as the magnet generating means. The sample table has the permanent magnets arranged as the driven magnet. Movement of the magnetic housing from the driving source of the sample table drive mechanism is turned into following working of the sample table by the non-contact coupling based on the magnetic force through the members forming the inside of the temperature-controlled chamber.
(20) The direct current electromagnets or the alternate current electromagnets can be used for the magnet generating means arranged on the magnetic housing. The strength of magnetic force can be controlled by using the electromagnets, thereby easily enabling to seek the magnetic force most suitable for transmitting the drive force to the sample table.
(21) The sample table can be worked without using the rotary drive force from the motor. In using the direct current electromagnets, the direct current electromagnets are fixed on the direct current electromagnets instead of the permanent magnets, and the magnetic poles of the direct current electromagnets are switched by the control assembly in order. In this way, the sample table can be followed by step motion. In this case, the sample table can be precisely positioned by raising the density within the fixed interval of the direct current electromagnets.
(22) The alternate current electromagnets can be also used instead of the direct current electromagnets. In this case, it can be made a direct drive motor type, which drives the permanent magnets located on the back side of the sample table as a movement terminal. Even if any driving method is adopted, the sample table drive mechanism can be repaired and maintained without influencing an environment in the temperature-controlled chamber even during testing or culturing by making the structure to be attached removably. Since there are heat insulating materials between the inside of the temperature-controlled chamber and the installation part of the sample table drive mechanism, it is possible to evade that the structure components of the sample table drive mechanism are influenced by the atmosphere such as high temperature and high humidity in the temperature-controlled chamber during testing or culturing.
(23) The sample table is put in the temperature-controlled chamber so as to be movable. The movement method may be rotary motion or linear motion to right and left for the small opening. Preferably, it is rotated around a perpendicular line which passes through the center of the temperature-controlled chamber.
(24) The sample table has rolling means or members having low frictional residence used as base material provided on the back side to surely follow the movement of the sample table drive mechanism. It is desirable that the permanent magnets located on the back side of the sample table are separated by a constant distance from the floor of the temperature-controlled chamber or covered with the members having low frictional resistance so as not to be rubbed. Similarly, in case the sample table drive mechanism has the permanent magnets or the direct current electromagnets, it is desirable to get rid of friction load by separating from the back side of the floor of the temperature-controlled chamber by a constant distance because these magnets move in themselves.
(25) The sample shelf is so constructed that the sample containers are vertically put on a plurality of shelves. The shelves may be laterally fixed in a plurality of rows, or may be separated by every vertical one line. In case of a disk-like sample table, the shelves are constructed so as to be integrated in a cylindrical shape along the lengthwise and the circumference or so as to be separated by every vertical one line. In the latter case, the disk-like sample table has portions for carrying in and out arranged in a circle for the outside. Although the shelves are put on the top or the side of the positioning tool provided on the sample table, they can be fixed with screws or springs so as not to cause misregistration.
(26) Since the large opening formed on one side of the temperature-controlled chamber is hardly opened and closed while the temperature-controlled chamber is driven, it is opened and closed with a door by hand control. Here, the door may be a hinge type or a male-female type, and mounted on the side or the ceiling. The door is preferably provided to the side of the temperature-controlled chamber, and may have a glass window fitted to observe the inside.
(27) The small opening is preferably provided just before an optional shelf not a uniformity place of the side of the temperature-controlled chamber because it is opened at the positions of a plurality of shelves. The small opening has the opening area enough size that the conveyance mechanism can carry in and out at least one sample container. The shielding plate of the small opening may be made in a well-known shape or type. However, generally, in case of the door having hinges at the upper and the lower, there is a risk of disturbing of the atmosphere in the temperature-controlled chamber by causing an air current according to working for opening-and-closing door and by aggregating the atmosphere in the temperature-controlled chamber with the atmosphere of the outside.

Therefore, there is no disorder of the air current according to the working for opening-and-closing by using shielding plates, which open and close with sliding vertically or laterally, as a type for opening-and-closing door. The shielding plates are arranged between the conveyance mechanism and the external wall of the temperature-controlled chamber in parallel to the travel means without a gap. Here, the number of shielding plates is the same as the shelves.
(28) The sample shelf has the shelves arranged at an equal interval in the height and the shielding plates may be corresponded to the shelves. For example, in putting a sample container having the size for two shelves on the sample shelf, a partition between the shelves is removed. The size of the shielding plate may be equal to the height of each shelf of the sample shelf or may not. However, when it is recognized that a big container is put on beforehand, the pass box or the dome must be the size enough to contain the big container.
(29) The conveyance mechanism comprises a finger, arm mechanism for advancing and backing the finger, and travel means for laterally or vertically moving the finger and the arm mechanism. The conveyance mechanism has a pass box having the minimum capacity necessary for carrying the sample container, being constructed so as not to have a harmful influence on the internal environment in carrying the sample container in and out. Although it is preferable that there is no opening between the pass box and the shielding plates, means for improving the tightness such as a packing etc. also can be provided.
(30) The conveyance mechanism is attached removably on the external wall adjacent to the small opening of the temperature-controlled chamber, thereby enabling to repair and maintain without affecting the environment in the temperature-controlled chamber even during testing or cultivating. Since the conveyance mechanism is mounted on the outside, the components of conveyance mechanism are not affected by the atmosphere of high temperature and high humidity in the temperature-controlled chamber.
(31) The conveyance mechanism has means for engaging with the shielding plates, opening and closing the shielding plates from the outside of the temperature-controlled chamber without a mechanism for opening and closing them. That is, the conveyance mechanism and the shielding plates are integrally interlocked by the engaging means, enabling to open and close the shielding plates by the travel means of a part of the conveyance mechanism without a mechanism exclusive for opening and closing. According to this, the conveyance mechanism has few components and simple structure, thereby enabling reduction in cost and improvement in reliability. As for the engaging means, mechanical connection due to bar members or connection due to magnetic force is considered.
(32) The shielding plates can be lined up by the slide-guides movably in the vertical direction or the lateral direction. The shielding plates can improve the tightness between the adjoining portions by forming the sections of the adjoining portions in curb or a plurality of straight lines which can be interfitted.
(33) To improve the tightness between the shielding plates and the external wall of the constant-temperature equipment, the shielding plates are made of members which are attracted by the magnetic force or provided with the member and besides, a magnet is provided at the position where contacts to the shielding plate on the external wall of the temperature-controlled chamber. According to this, the shielding plate and the external wall of the temperature-controlled chamber are adhered by the magnetic force, thereby enabling to improve the tightness.
(34) Pulse motors such as a stepping motor and a servomotor are preferably used as a motor for the conveyance mechanism or the sample table drive mechanism. A control device is provided to automatically control these motors and to control the environmental conditions in the temperature-controlled chamber.
(35) It is preferable that there are no projections such as a rotating axis and a fixed shelf in the temperature-controlled chamber of the constant-temperature equipment. Further, it is preferable that the inside of the temperature-controlled chamber is smooth so as to be easily cleaned. However, it is possible to form holes for supplying or discharging gasses such as humidity air for controlling the environmental conditions, oxygen, nitrogen and carbon dioxide, and to provide with sensors for the temperature, the humidity and the concentration of various gasses. In this case, the inside may be constructed so as not to be influenced by the outside environment by making it positive pressure.
(36) The well-known technology such as a sump for humidification and a mechanism for de-dew-condensation, can be used in the temperature-controlled chamber. The temperature-controlled chamber can pool demineralized water because the floor is flat without mechanical components. In addition, the humidity during testing and culturing can be maintained by forming the sump out of concaves composed of smooth curved plane or by placing vats at four corners of the floor of the temperature- controlled chamber.
(37) Although the sample table is moved with non-contact state by the magnetic coupling in the above-mentioned examples, it can be moved by mechanical constituents such as a shaft which passes through from a motor installed on the outside of the temperature-controlled chamber into the temperature-controlled chamber, as shown in patent literatures 1, 2.
(38) As a detachable pierce, magnetic coupling for absorbing the shielding plate with the electromagnets can be used instead of mechanical coupling such as the engaging pin.

According to these embodiments, the following effects can be obtained.

As shown in Fig. 2, because all of electrical and mechanical structures such as the sample shelf 4, the sample table 5, the sample table drive mechanism 6, the conveyance mechanism 11 and the travel means 12 are removed from the temperature-controlled chamber 15, dry sterilization at high temperature, which was difficult with the conventional automatic incubators, can be carried out all of the structures in the temperature-controlled chamber 15. In this case, the components do not break down at the high temperature at the time of the dry sterilization by removing the electrical and mechanical structures.

Since the temperature-controlled chamber has no irregularities due to the electrical and mechanical structures, it is possible to easily carry out wiping before and after the sterilization and carry out highly reliable sterilization. Therefore, it is possible to carry out highly reliable culturing and testing. Since the sample shelf 4 and the sample table 5 can be easily removed from the temperature-controlled chamber 15, they can be individually sterilized and washed in sterilized liquid.

Since the temperature-controlled chamber 15 has no electrical and mechanical structures for the driving portion and has the insulating materials 22 put along the portion connected to the sample table drive mechanism 6, the driving portion is not affected by the atmosphere in the high temperature and humidity. Therefore, the driving portion hardly breaks down, thereby enabling to avoid from suspensions or interruptions of the culturing and testing caused by the trouble.

If the electrical and mechanical structures should break down during the culturing and testing, the sample table drive mechanism 6 or the conveyance mechanism 11 can be removed and repaired because the sample table drive mechanism 6, the conveyance mechanism 11 and the temperature-controlled chamber 15 are completely separated one another. Therefore, it is possible to avoid from the suspensions of the culturing and testing. In addition, good culture environment can be maintained by providing with means for keeping humidity during the culturing and testing.

Since the temperature-controlled chamber 15 has the plane bottom without mechanical components, it is possible to pool pure water therein to maintain the humidity during the culturing and testing.

Since the conveyance mechanism 11 has the pass box having the minimum capacity necessary for carrying the container, the internal environment, which is kept in constant temperature and humidity, is not affected in carrying in and out the container. Therefore, it is possible to maintain stably culturing and testing environment.

Since the slide type is adopted for opening and closing the shielding plates and ones except the required openings are always closed, unnecessary air-flow is not generated in the temperature-controlled chamber at the time of the opening and closing. Therefore, it is possible to maintain stable culturing and testing environment.

## Claims

1. Constant-temperature equipment comprising:
a temperature-controlled chamber (15) having a sample shelf (4) and an opening portion (8), said sample shelf (4) being provided to an inside and having a plurality of shelves, and said opening portion (8) being opened for an outside at a position corresponding to each shelf of the sample shelf (4);
a slide frame (10) provided along a side edge of the opening portion (8);
a plurality of shielding plates (9) for closing the opening portion (8) and guided to the slide frame (10), each of said shielding plates (9) closing or opening an interval with adjoining shielding plate while being guided to the slide frame (10); and
conveyance mechanism (11) having a detachable piece for removably attaching to the shielding plates (9), wherein the conveyance mechanism (11) is movable on the shielding plates (9) along the side edge of the opening portion and attached removably on an external wall (13) of the constant-temperature equipment to a position facing the shielding plates (9).

2. Constant-temperature equipment according to claim 1, further comprising a pass box (53) having a finger (33), arm mechanisms for advancing and backing the finger (33), and slewing mechanism for slewing the finger (33) and the arm mechanism, wherein the detachable piece is mounted on an outside of the pass box (53).

3. Constant-temperature equipment according to claim 1, further having a dorm (90) in the pass box (53), said dorm (90) having an opening at one side and rotating in a body with the slewing mechanism, wherein the finger and the arm mechanisms are located in the dorm.

4. Constant-temperature equipment according to claim 2, wherein the opening portion (8) forms a lengthwise opening, the slide frame (10) guides the shielding plates (9) vertically, the detachable piece is engaged with any of the shielding plates (9), the conveyance mechanism (11) moves upward, and therefore, a space within the pass box (53) and a space within the sample shelf (4) are connected by lifting up upper shielding plates (9) including the engaged shielding plate (9).

5. Constant-temperature equipment according to claim 1, wherein the shielding plates (9) are provided correspondent to the shelves of the sample shelf (4).

6. Constant-temperature equipment according to claim 1, wherein the shielding plates (9) have engaging holes or brackets and the detachable piece is engaged to an engaging hole or a bracket.

7. Constant-temperature equipment according to claim 1, wherein a bracket for fitting and removing the conveyance mechanism is provided on the shielding plates.

8. Constant-temperature equipment according to claim 1, wherein the temperature-controlled chamber has a sample table (5), on which the sample shelf (4) is loaded, put on a bottom, and sample table drive mechanism (6) for moving the sample table (5) by magnetic coupling through the bottom plate from the outside.

9. Constant-temperature equipment according to claim 8, wherein the sample table (5) has a rolling body or a low frictional member provided on the bottom.

10. Constant-temperature equipment according to claim 8, wherein the sample table drive mechanism (6) has magnetic driving means, the sample table (5) has driven magnets (30) arranged opposite to the magnetic generating means through the bottom plate, following the magnetic generating means due to magnetic coupling between the magnetic generating means and the driven magnets (30).

11. Constant-temperature equipment according to claim 10, wherein the driven magnets (30) are permanent magnets and the sample table drive mechanism has permanent magnets for magnetically coupling with the driven magnets (30).

12. Constant-temperature equipment according to claim 10, the driven magnets (30) are permanent magnets and the sample table drive mechanism has electromagnets for magnetically coupling with the driven magnets (30).

13. Constant-temperature equipment according to claim 12, wherein the electromagnets are to generate a shifting magnetic field.

14. Constant-temperature equipment according to claim 10, wherein the sample table drive mechanism has a turntable and either one kind of permanent magnets or direct current electromagnets arranged to a center of the turntable, and the sample table (5) is rotated.

15. Constant-temperature equipment according to claim 1, wherein the temperature-controlled chamber (15) has a sample table (5), on which the sample shelf (4) is loaded, loaded on a bottom, and sample table drive mechanism for moving the sample table (5) from an outside of the bottom.

## Patentansprüche

1. Vorrichtung zur Temperaturkonstanthaltung, enthaltend:
eine temperaturkontrollierte Kammer (15) mit einem Probenregal (4) und einem Öffnungsbereich (8), wobei das Probenregal (4) innenliegend bereitgestellt ist und eine Mehrzahl von Ablagen aufweist, und der Öffnungsbereich (8) nach außen an einer Position offen ist, die jeder Ablage des Probenregals (4) entspricht;
einen Gleitrahmen (10), der entlang eines seitlichen Rands des Öffnungsbereichs (8) bereitgestellt ist;
eine Mehrzahl von Abschirmungsplatten (9) zum Schließen des Öffnungsbereichs (8) und an dem Gleitrahmen (10) gerührt, wobei jede Abschirmungsplatte (9) einen Abschnitt schließt oder öffnet mit einer angrenzenden Abschirmungsplatte, während einer Führung auf dem Gleitrahmen (10); und
einen Transportmechanismus (11), der ein abnehmbares Teil aufweist zum entfernbaren Anbringen an den Abschirmungsplatten (9), wobei der Transportmechanismus (11) auf den Abschirmungsplatten (9) entlang des seitlichen Rands des Öffnungsbereichs bewegbar und entfernbar an einer äußeren Wand (13) der Vorrichtung zur Temperaturkonstanthaltung an einer Position angebracht ist, die zu den Abschirmungsplatten (9) weist.

2. Vorrichtung zur Temperaturkonstanthaltung nach Anspruch 1, ferner enthaltend ein Durchgangskasten (53) mit einem Finger (33), einem Armmechanismus zum Vor- und Zurückfahren des Fingers (33), und einem Schwenkmechanismus zum Schwenken des Fingers (33) und des Armmechanismus, wobei das abnehmbare Teil an einer Außenseite des Durchgangskastens (53) montiert ist.

3. Vorrichtung zur Temperaturkonstanthaltung nach Anspruch 1, ferner enthaltend einen Ring (90) in dem Durchgangskasten (53), wobei der Ring (90) an einer Seite eine Öffnung aufweist und gemeinsam mit dem Schwenkmechanismus dreht, wobei der Finger und der Armmechanismus sich in dem Ring befinden.

4. Vorrichtung zur Temperaturkonstanthaltung nach Anspruch 2, bei der der Öffnungsbereich (8) eine Längsöffnung bildet, der Gleitrahmen (10) die Abschirmungsplatten (9) vertikal führt, das abnehmbare Teil in Eingriff ist mit irgendeiner der Abschirmungsplatten (9), der Transportmechanismus (11) sich aufwärts bewegt, und folglich ein Raum innerhalb des Durchgangskastens (53) und ein Raum innerhalb des Probenregals (4) verbunden werden, indem obere Abschirmungsplatten (9), einschließlich die eingegriffene Abschirmungsplatte (9), hochgehoben werden.

5. Vorrichtung zur Konstanttemperaturhaltung nach Anspruch 1, bei der die Abschirmungsplatten (9) entsprechend den Ablagen des Probenregals (4) bereitgestellt sind.

6. Vorrichtung zur Konstanttemperaturhaltung nach Anspruch 1, bei der die Abschirmungsplatten (9) Eingriffslöcher oder Klammern haben und das abnehmbare Teil mit einem Eingriffsloch oder einer Klammer in Eingriff ist.

7. Vorrichtung zur Konstanttemperaturhaltung nach Anspruch 1, bei der eine Klammer zum Befestigen und Entfernen des Transportmechanismus auf den Abschirmungsplatten bereitgestellt ist.

8. Vorrichtung zur Konstanttemperaturhaltung nach Anspruch 1, bei der die temperaturkontrollierte Kammer einen Probentisch (5) hat, auf dem das Probenregal (4) aufgeladen ist, auf einem Boden, und einen Probentischantriebsmechanismus (6) zum Bewegen des Probentisches (5) durch Magnetkopplung über die Bodenplatte von außen.

9. Vorrichtung zur Konstanttemperaturhaltung nach Anspruch 8, bei der der Probentisch (5) einen Rollkörper oder ein unteres Reibbauteil hat, die auf dem Boden bereitgestellt sind.

10. Vorrichtung zur Konstanttemperaturhaltung nach Anspruch 8, bei der der Probentischantriebsmechanismus (6) ein Magnetantriebsmittel hat, der Probentisch (5) angetriebene Magneten (30) hat, die dem Magneterzeugungsmittel über die Bodenplatte gegenüberliegend angeordnet sind und dem Magneterzeugungsmittel aufgrund der Magnetkopplung zwischen dem Magneterzeugungsmittel und den angetriebenen Magneten (30) folgen.

11. Vorrichtung zur Konstanttemperaturhaltung nach Anspruch 10, bei der die angetriebenen Magnete (30) Dauermagnete sind, und der Probentischantriebsmechanismus Dauermagnete hat zur magnetischen Kopplung mit den angetriebenen Magneten (30).

12. Vorrichtung zur Konstanttemperaturhaltung nach Anspruch 10, bei der die angetriebenen Magnete (30) Dauermagnete sind, und der Probentischantriebsmechanismus Elektromagnete hat zur magnetischen Kopplung mit den angetriebenen Magneten (30).

13. Vorrichtung zur Konstanttemperaturhaltung nach Anspruch 12, bei der die Elektromagnete dazu dienen eine Magnetfeldverschiebung zu erzeugen.

14. Vorrichtung zur Konstanttemperaturhaltung nach Anspruch 10, bei der der Probentischantriebsmechanismus eine Drehscheibe hat, und die Dauermagnete oder die Gleichstromelektromagnete an einem Zentrum der Drehscheibe angeordnet sind, und der Probentisch (5) gedreht wird.

15. Vorrichtung zur Konstanttemperaturhaltung nach Anspruch 1, bei der die temperaturkontrollierte Kammer (15) einen Probentisch (5) hat, auf dem das Probenregal (4) aufgeladen ist, aufgeladen auf einen Boden, und einen Probentischantriebsmechanismus zum Bewegen des Probentisches (5) von außerhalb des Bodens aus.

## Revendications

1. Equipement à température constante, comprenant :
une chambre (15) à température contrôlée ayant une étagère à échantillons (4) et un portion d'ouverture (8), laquelle étagère à échantillons (4) est prévue pour un intérieur et comprend une pluralité d'étagères, et laquelle portion d'ouverture (8) s'ouvrant d'un extérieur à une position correspondant à chaque étagère de l'étagère à échantillons (4) ;
un châssis à glissement (10) prévu le long du côté de la portion d'ouverture (8) ;
une pluralité de tôles de blindage (9) pour fermer la portion d'ouverture (8) et guide vers le châssis à glissement (10), chacune desdites tôles de blindage (9) fermant et ouvrant un intervalle avec des tôles de blindages adjointes en étant guidés vers le châssis à glissement (10) ; et
un mécanisme de transport (11) ayant une pièce détachable s'attachant aux tôles de blindage (9) de manière amovible, où le mécanisme de transport (11) est déplaçable sur les tôles de blindage (9) le long du côté de la portion d'ouverture et attaché de manière amovible à un mur externe (13) de l'équipement à température constante dans une position face aux tôles de blindage (9).

2. Equipement à température constante selon la revendication 1, comprenant en outre un sas (53) ayant un doigt (33), des mécanismes de bras pour faire avancer et reculer le doigt (33), et un mécanisme d'orientation pour orienter le doigt (33) et le mécanisme de bras, où la pièce détachable est montée sur un côté extérieur du sas (53).

3. Equipement à température constante selon la revendication 1, comprenant en outre un logement (90) dans le sas (53), lequel logement (90) ayant une ouverture d'un côté et pivotant dans un corps avec le mécanisme d'orientation, où le doigt et le mécanisme de bras sont localisés dans le logement.

4. Equipement à température constante selon la revendication 2, dans lequel la portion d'ouverture (8) forme une ouverture dans le sens de la longueur, le châssis à glissement (10) guide les tôles de blindage (9) verticalement, la pièce détachable est engagée avec une quelconque des tôles de blindage (9), le mécanisme de transport (11) se déplace vers le haut, et pour cela, un espace dans le sas (53) et un espace dans l'étagère à échantillons (4) sont connectés en soulevant les tôles de blindage (9) supérieures comprenant la tôle de blindage (9) engagée.

5. Equipement à température constante selon la revendication 1, dans lequel les tôles de blindage (9) sont prévues correspondant aux étagères de l'étagère à échantillons (4).

6. Equipement à température constante selon la revendication 1, dans lequel les tôles de blindage (9) ont des trous d'engagement ou des crochets et la pièce détachable est engagée à un trou d'engagement ou a un crochet.

7. Equipement à température constante selon la revendication 1, dans lequel un crochet est prévu sur les tôles de blindage pour ajuster et retirer le mécanisme de transport.

8. Equipement à température constante selon la revendication 1, dans lequel la chambre à température contrôlée a une table d'échantillons (5), sur laquelle l'étagère à échantillons (4) est chargée, mise au fond, et un mécanisme de conduite (6) de la table d'échantillons afin de déplacer la table d'échantillons (5) par couplage magnétique du plateau de fond vers l'extérieur.

9. Equipement à température constante selon la revendication 8, dans lequel la table d'échantillons (5) a un corps roulant ou un organe à basse friction prévu dans son fond.

10. Equipement à température constante selon la revendication 8, dans lequel le mécanisme de conduite (6) de la table d'échantillons possède des moyens de propulsion magnétique, la table d'échantillons (5) possède des aimants de propulsion (30), disposés de façon opposés au moyens de propulsion magnétique au travers du plateau de fond, poursuivant le moyen de génération magnétique dus au couplage magnétique entre le moyen de génération magnétique et les aimants (30) de propulsion.

11. Equipement à température constante selon la revendication 10, dans lequel les aimants (30) de propulsion sont des aimants permanents et le mécanisme de propulsion (6) de la table d'échantillons possède des aimants permanents pour le couplage magnétique avec les aimants (30) de propulsion.

12. Equipement à température constante selon la revendications 10, les aimants (30) de propulsion sont des aimants permanents et le mécanisme de propulsion de la table d'échantillons a des électro-aimants pour le couplage magnétique avec les aimants (30) de propulsion.

13. Equipement à température constante selon la revendication 12, dans lequel les électroaimants sont prévus pour générer un champs magnétique de déplacement.

14. Equipement à température constante selon la revendication 10, dans lequel le mécanisme de propulsion de la table d'échantillons a une table tournante et l'un et l'un quelconque des aimants permanents ou des électro-aimants à courant direct arrangés à un centre de la table tournante, et la table d'échantillons (5) est rotative.

15. Equipement à température constante selon la revendication 1, dans laquelle la chambre à température contrôlée (15) a une table d'échantillons (5), sur laquelle l'étagère à échantillons (4) est chargée, chargée sur le fond, et le mécanisme de propulsion de la table d'échantillons pour déplacer la table d'échantillons (5) d'un côté extérieur du fond.
